# EUROPEAN PATENT SPECIFICATION

(11) **EP 4 285 893 B1**
(45) Date of publication and mention of the grant of the patent: **29.04.2026**
(21) Application number: 23174753.6
(22) Date of filing: 23.05.2023
(51) Int. Cl.: A61K 9/20, A61K 47/12

(54) **A COMBINATION COMPRISING EMPAGLIFLOZIN AND METFORMIN HYDROCHLORIDE**
KOMBINATION MIT EMPAGLIFLOZIN UND METFORMINHYDROCHLORID
COMBINAISON COMPRENANT DE L'EMPAGLIFLOZINE ET DE L'HYDROCHLORURE DE METFORMINE

(30) Priority: 31.05.2022 TR 202208871
(43) Date of publication of application: 06.12.2023
(73) Proprietor: Sanovel Ilac Sanayi ve Ticaret A.S., Sisli - Istanbul (TR)
(72) Inventor: GULER, Tolga, Istanbul (TR); ARMUT, Merve, Istanbul (TR); SUNEL, Fatih, Istanbul (TR); PEHLIVAN AKALIN, Nur, Istanbul (TR)
(74) Representative: Genç Ilhan, Oznur

(56) References cited:
- WO-A1-2020/058095
- WO-A1-2021/123165
- CN-A- 114 404 436

## Description

### Field of the Invention

The present invention relates to a film coated tablet comprising metformin hydrochloride and empagliflozin wherein comprises sodium stearyl fumarate as a lubricant, as defined by the claims. The tablet provides pharmacotechnical properties and the desired dissolution profile. The present invention also relates to a simple, rapid, cost effective, time-saving and industrially convenient method of preparing the tablet.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2: Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose. In Type 2 diabetes, the pancreas makes insulin, but it either doesn't produce enough, or the insulin does not work properly. This diabetes occurs most often in people who are over 40 years old and overweighed. Type 2 diabetes may sometimes be controlled with a combination of diet, weight management, and exercise. However, treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether and chloroform. Oral doses of metformin are generally recommended in the range of 500 to 2500 mg a day and a single dose may vary from 500 to 850 mg.

It is used singly or in combination with sulfonylureas, alpha-glucosidase inhibitors, or insulin.

The chemical name of metformin hydrochloride is 1,1-dimethylbiguanide hydrochloride, has the following chemical structure of Formula I.

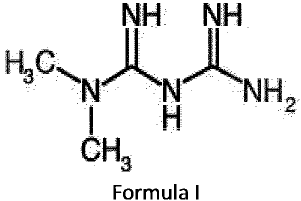

Empagliflozin is a known SGLT2 inhibitor that is described for the treatment or improvement in glycemic control in patients with type 2 diabetes mellitus. The chemical name of empagliflozin is 1 - chloro-4-(3-D-glucopyranos-1 -yl)- 2-[4-((S)-tetrahydrofuran-3-yloxy)-benzyl]-benzene and its chemical structure is shown in the Formula II.

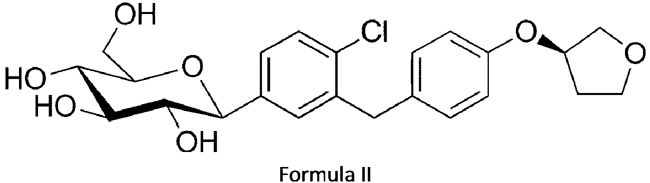

Combination product of empagliflozin and metformin hydrochloride is marketed under the trademark Synjardy^{®}. The combination is to help control blood glucose in people with T2D. Empagliflozin, a sodium glucose co-transporter-2 (SGLT2) inhibitor, removes excess glucose through the urine by blocking glucose re-absorption in the kidney.

Active ingredients have some disadvantages in the formulation and process. The main problem encountered when preparing formulations comprising empagliflozin is low solubility, leading to difficulties with disintegration and dissolution times. Furthermore, metformin is a very poorly compressible active substance and metformin presents in high amounts in a composition. This causes some problems for examples; homogeneity, flowability and dissolution profile.

WO2011039337 (A1) application discloses pharmaceutical compositions comprising fixed dose combinations of a SGLT-2 inhibitor drug and a partner drug, processes for the preparation thereof, and their use to treat certain diseases.

CN104586834 (A) application discloses a pharmaceutical composition of empagliflozin and metformin, a preparation method and application thereof. The composition comprises the following components: i.) empagliflozin; ii.) metformin hydrochloride; and one or more fillers; one or more adhesives; one or more flow aids and one or more lubricants.

In the prior art, there are also several patents which disclose empagliflozin and metformin hydrochloride in oral pharmaceutical dosage forms. However, because of the dissolution problem of empagliflozin, and the poorly compressible of metformin, an effective formulation and method has not been disclosed.

There still remains a need in the art to provide an improved a film coated tablet comprising empagliflozin and metformin hydrochloride having high solubility, excellent pharmacomechanic properties and accordingly a high bioavailability and long term stability.

WO 2021/123165 A1 discloses a solid pharmaceutical dosage form comprising an amorphous solid solution of empagliflozin with at least one polymer, and exemplifies a film coated tablet comprising empagliflozin in combination with metformin hydrochloride and magnesium stearate as lubricant. WO 2021/123165 A1 focuses on providing pharmaceutical compositions comprising empagliflozin, which are which are physically and chemically stable, have improved dissolution profile and are prepared by an economical process suitable for use on a commercial scale.

WO 2020/058095 A1 relates to stable pharmaceutical compositions of empagliflozin, in the form of immediate release tablets and to a process for the manufacture of said stable pharmaceutical compositions. The aim of WO 2020/058095 A1 is to provide a pharmaceutical composition with a good dissolution profile that enables a high bioavailability of empagliflozin in a patient, while it avoids or reduces filming and sticking during the manufacturing process of the composition.

CN 114 404 436 A belongs to the field of medicine, in particular to a metformin empagliflozin composition and preparation method thereof.

### Detailed Description of the Invention

The main object of the present invention is to provide a film coated tablet comprising empagliflozin and metformin hydrochloride with excellent physicochemical properties, such as compressibility, flowability, homogeneity, and content uniformity which overcomes the above-described problems in the prior art and have additive advantages over them.

Another object of the present invention is to provide a film coated tablet comprising empagliflozin and metformin hydrochloride with having the desired level of dissolution rate and high stability. Another object of the present invention is to provide a process for preparing a film coated tablet comprising empagliflozin and metformin hydrochloride. The process is a simple, rapid, cost effective, time-saving, and industrially convenient method.

The main problem encountered when preparing formulations comprising empagliflozin is low solubility, leading to difficulties with disintegration and dissolution times. Furthermore, metformin is a very poorly compressible active substance and metformin presents in high amounts in a composition. This causes some problems for examples; homogeneity, flowability and dissolution profile. Therefore, the excipients and process steps used are very important. We found that especially for flowability using sodium stearyl fumarate eliminated the problems.

Flowability is an important parameter in tablet formulation. It can be a problem especially in formulations containing a high amount of active substance. In a formulation, the problem with flowability negatively affects the content uniformity, and the problem with the content uniformity negatively affects the dissolution profile. In this formulation, the selected excipients are very important since both metformin HCl has a high amount and metformin already has flowability and compressibility problems. We have found in this invention that a particularly well-chosen lubricant overcomes these problems.

According to an embodiment of the present invention, a film coated tablet comprises metformin hydrochloride and empagliflozin comprising sodium stearyl fumarate as a lubricant, as defined by the claims. The film coated tablet is obtained which provides good flowability of the powder/granules. This choice of lubricant eliminates the disadvantages of both active agents and provides an effective tablet formulation.

According to an embodiment of the present invention, the amount of sodium stearyl fumarate is 0.1% to 5.0% by weight in the total composition.

According to an embodiment of the present invention, the amount of metformin hydrochloride is 70.0% to 90.0% by weight in the total composition. Preferably, the amount of metformin hydrochloride is 80.0% to 90.0% by weight in the total composition.

According to an embodiment of the present invention, the amount of empagliflozin is 0.1% to 5.0% by weight in the total composition. Preferably, the amount of empagliflozin is 0.3% to 2.5% by weight in the total composition.

Encountered while developing formulations is the flowability-problem and compressibility of metformin HCl, which makes the production difficult. Using at least one diluent, as defined by the claims, helps to overcome this problem.

According to an embodiment of the present invention, the film coated tablet further comprises at least one diluent and at least one binder and at least one glidant.

Suitable diluents are selected from the group comprising corn starch, which is according to the claims, but can further relate to microcrystalline cellulose, lactose, anhydrous lactose, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

According to the present invention, the diluent is corn starch. The filler provides flowability and compressibility of the powder mixture.

According to an embodiment of the present invention, the amount of diluents is 1.0% to 12.0% by weight in the total composition. Preferably, the amount of diluents is 2.0% to 8.0% by weight in the total composition.

Suitable binders are selected from the group comprising hydroxypropyl cellulose, hydroxypropyl methylcellulose, pregelatinized starch, polyvinylpyrrolidone, carboxymethylcellulose sodium, cellulose acetate phthalate, chitosan, dextrose, ethylcellulose, glyceryl behenate, hydroxyethyl cellulose, hydroxyethylmethyl cellulose, hydroxypropyl starch, magnesium aluminum silicate, poloxamer, polycarbophil, polydextrose, polyethylene oxide, polymethacrylates, polyoxyethilene-alkyl ethers or mixtures thereof.

According to an embodiment of the present invention, the binder is hydroxypropyl methyl cellulose or hydroxypropyl cellulose.

According to an embodiment of the present invention, the amount of binders is 2.0% to 15.0% by weight in the total composition. Preferably, the amount of binders is 2.0% to 10.0% by weight in the total composition.

Suitable glidants are selected from the group comprising anhydrous colloidal silicon dioxide, sodium stearyl fumarate, magnesium oxide, silicone dioxide, talc, polyethylene glycol, stearic acid, aluminum silicate, magnesium silicate or mixtures thereof.

According to an embodiment of the present invention, the glidant is anhydrous colloidal silicon dioxide.

According to an embodiment of the present invention, the amount of glidants is 0.1% to 6.0% by weight in the total composition. The amount of glidants used helps to provide the desired flowability and compressibility of tablet.

According to an embodiment of the present invention, the film coated tablet is coated with at least one film coating agent.

Suitable film coating agents are selected from the group comprising polymethacrylates, hydroxypropyl methylcellulose, lactose monohydrate, talc, hydroxypropyl cellulose, polyvinyl alcohol (PVA), polyethylene glycol (PEG), glycerin, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), iron oxide yellow, iron oxides, all kinds of Opadry^{®}, pigments, dyes, titanium dioxide, coloring agent or mixtures thereof.

According to an embodiment of the present invention, the film coated tablet comprises;
a) Metformin HCl
b) Empagliflozin
c) Corn starch
d) Hydroxypropyl Cellulose or hydroxypropyl methylcellulose
e) Anhydrous colloidal silicon dioxide
f) Sodium stearyl fumarate.

The film coated tablet of the present invention is prepared wet granulation.

According to one embodiment of the present invention, a process for the preparation of the film coated tablet comprises the following steps:
a) Mixing Metformin HCl, empagliflozin, corn starch and hydroxypropyl Cellulose or hydroxypropyl methylcellulose,
b) Granulating the mixture with a solvent,
c) Drying the wet granules,
d) Adding anhydrous colloidal silicon dioxide and sodium stearyl fumarate and then mixing,
e) Compressing the mixture into the tablet,
f) Coating the tablets with film coating agent.

According to this embodiment of the present invention, a solvent is used at wet granulation.

Suitable solvents are selected from the group comprising pure water, dichloromethane, 0.1N HCl, methanol, ethanol, isopropyl alcohol, benzyl alcohol, propylene glycol, polyethylene glycol, cyclomethicone or mixtures thereof. Preferably, the solvent is water.

In this present invention, a desired flowability and compressibility and a desired content uniformity of the film coated tablet is obtained and it has a simple and low-cost preparation process, in favor of industrial production.

### Example 1: The tablet formulation

| **Ingredients** | **% by weight** |
|---|---|
| Metformin HCl | 70.0 - 90.0 |
| Empagliflozin | 0.1 - 5 |
| Corn starch | 1.0 - 12.0 |
| Hydroxypropyl Cellulose or hydroxypropyl methylcellulose | 2.0 - 15.0 |
| Anhydrous colloidal silicon dioxide | 0.1 - 6.0 |
| Sodium stearyl fumarate | 0.1 - 5.0 |
| Coating | 0.5 - 4.0 |
| **TOTAL** | **100** |

A process for example 1;
a) Sieving metformin HCl through 2 mm,
b) Mixing Metformin HCl, empagliflozin, corn starch and hydroxypropyl Cellulose or hydroxypropyl methylcellulose,
c) Granulating the mixture with a solvent (for example; pure water)
d) Sieving the wet granule through 8 mm,
e) Drying the wet granules at 50 °C and sieving through 1.5 mm,
f) Adding anhydrous colloidal silicon dioxide and sodium stearyl fumarate and then mixing,
g) Compressing the mixture into the tablet,
h) Coating the tablets with film coating agent.

## Claims

1. A film coated tablet comprises metformin hydrochloride and empagliflozin comprising sodium stearyl fumarate as a lubricant and corn starch as a diluent.

2. The film coated tablet according to claim 1, wherein the amount of sodium stearyl fumarate is 0.1% to 5.0% by weight in the total composition

3. The film coated tablet according to claim 1, wherein further comprises at least one binder and at least one glidant.

4. The film coated tablet according to claim 1, wherein the amount of diluents is 1.0% to 12.0% by weight in the total composition.

5. The film coated tablet according to claim 3, wherein binders are selected from the group comprising hydroxypropyl cellulose, hydroxypropyl methylcellulose, pregelatinized starch, polyvinylpyrrolidone, carboxymethylcellulose sodium, cellulose acetate phthalate, chitosan, dextrose, ethylcellulose, glyceryl behenate, hydroxyethyl cellulose, hydroxyethylmethyl cellulose, hydroxypropyl starch, magnesium aluminum silicate, poloxamer, polycarbophil, polydextrose, polyethylene oxide, polymethacrylates, polyoxyethilene-alkyl ethers or mixtures thereof.

6. The film coated tablet according to claim 3, wherein the binder is hydroxypropyl methyl cellulose or hydroxypropyl cellulose.

7. The film coated tablet according to claim 3, wherein the amount of binders is 2.0% to 15.0% by weight in the total composition.

8. The film coated tablet according to claim 3, wherein glidants are selected from the group comprising anhydrous colloidal silicon dioxide, sodium stearyl fumarate, magnesium oxide, silicone dioxide, talc, polyethylene glycol, stearic acid, aluminum silicate, magnesium silicate or mixtures thereof.

9. The film coated tablet according to claim 3, wherein the glidant is anhydrous colloidal silicon dioxide.

10. The film coated tablet according to claim 1, wherein the film coated tablet comprises;
a) Metformin HCl
b) Empagliflozin
c) Corn starch
d) Hydroxypropyl Cellulose or hydroxypropyl methylcellulose
e) Anhydrous colloidal silicon dioxide
f) Sodium stearyl fumarate.

11. A process for the preparation of the film coated tablet comprises the following steps:
a) Mixing Metformin HCl, empagliflozin, corn starch, and hydroxypropyl Cellulose or hydroxypropyl methylcellulose,
b) Granulating the mixture with a solvent,
c) Drying the wet granules,
d) Adding anhydrous colloidal silicon dioxide and sodium stearyl fumarate and then mixing,
e) Compressing the mixture into the tablet,
f) Coating the tablets with film coating agent.

12. The process according to claim 11, wherein solvent is water.

## Patentansprüche

1. Filmtablette, umfassend Metforminhydrochlorid und Empagliflozin, umfassend Natriumstearylfumarat als Gleitmittel und Maisstärke als Verdünnungsmittel.

2. Filmtablette nach Anspruch 1, wobei die Menge an Natriumstearylfumarat 0,1 bis 5,0 Gew.-% der Gesamtzusammensetzung beträgt.

3. Filmtablette nach Anspruch 1, wobei sie ferner mindestens ein Bindemittel und mindestens ein Gleitmittel enthält.

4. Filmtablette nach Anspruch 1, wobei die Menge an Verdünnungsmitteln 1,0 bis 12,0 Gew.-% der Gesamtzusammensetzung beträgt.

5. Filmtablette nach Anspruch 3, wobei die Bindemittel aus der Gruppe ausgewählt sind bestehend aus Hydroxypropylcellulose, Hydroxypropylmethylcellulose, vorgelatinierter Stärke, Polyvinylpyrrolidon, Carboxymethylcellulose-Natrium, Celluloseacetatphthalat, Chitosan, Dextrose, Ethylcellulose, Glycerylbehenat, Hydroxyethylcellulose, Hydroxyethylmethyl Cellulose, Hydroxypropylstärke, Magnesiumaluminiumsilikat, Poloxamer, Polycarbophil, Polydextrose, Polyethylenoxid, Polymethacrylate, Polyoxyethylenalkylether oder Mischungen davon.

6. Filmtablette nach Anspruch 3, wobei das Bindemittel Hydroxypropylmethylcellulose Cellulose oder Hydroxypropylcellulose ist.

7. Filmtablette nach Anspruch 3, wobei die Menge an Bindemitteln 2,0 bis 15,0 Gew.-% der Gesamtzusammensetzung beträgt.

8. Filmtablette nach Anspruch 3, wobei die Gleitmittel aus der Gruppe ausgewählt sind aus wasserfreiem kolloidalem Siliciumdioxid, Natriumstearylfumarat, Magnesiumoxid, Siliciumdioxid, Talk, Polyethylenglykol, Stearinsäure, Aluminiumsilikat, Magnesiumsilikat oder Mischungen davon.

9. Filmtablette nach Anspruch 3, wobei das Gleitmittel wasserfreies kolloidales Siliciumdioxid ist.

10. Filmtablette nach Anspruch 1, wobei die Filmtablette umfasst:
a) Metformin-HCl
b) Empagliflozin
c) Maisstärke
d) Hydroxypropylcellulose oder Hydroxypropylmethylcellulose
e) wasserfreies kolloidales Siliciumdioxid
f) Natriumstearylfumarat.

11. Ein Verfahren zur Herstellung der Filmtablette umfasst die folgenden Schritte:
a) Mischen von Metformin-HCl, Empagliflozin, Maisstärke und Hydroxypropylcellulose oder Hydroxypropylmethylcellulose,
b) Granulieren der Mischung mit einem Lösungsmittel,
c) Trocknen der feuchten Granulate,
d) Zugabe von wasserfreiem kolloidalem Siliciumdioxid und Natriumstearylfumarat und anschließendes Mischen,
e) Verpressen der Mischung zu Tabletten,
f) Überziehen der Tabletten mit einem Filmüberzugsmittel.

12. Verfahren nach Anspruch 11, wobei das Lösungsmittel Wasser ist.

## Revendications

1. - Comprimé pelliculé comprenant du chlorhydrate de metformine et de l'empagliflozine, comprenant du fumarate de sodium et de stéaryle comme lubrifiant et de l'amidon de maïs comme diluant.

2. - Comprimé pelliculé selon la revendication 1, dans lequel la quantité de fumarate de sodium et de stéaryle est de 0,1 % à 5,0 % en poids dans la composition totale.

3. - Comprimé pelliculé selon la revendication 1, comprenant en outre au moins un liant et au moins un agent de glissement.

4. - Comprimé pelliculé selon la revendication 1, dans lequel la quantité de diluants est de 1,0 % à 12,0 % en poids dans la composition totale.

5. - Comprimé pelliculé selon la revendication 3, dans lequel les liants sont choisis dans le groupe comprenant l'hydroxypropyl cellulose, l'hydroxypropyl méthylcellulose, l'amidon prégélatinisé, la polyvinylpyrrolidone, la carboxyméthylcellulose sodique, l'acétate phtalate de cellulose, le chitosan, le dextrose, l'éthylcellulose, le béhénate de glycéryle, l'hydroxyéthyl cellulose, l'hydroxyéthylméthyl cellulose, l'hydroxypropyl amidon, le silicate d'aluminium et de magnésium, un poloxamère, un polycarbophile, un polydextrose, le poly (oxyde d'éthylène), les polyméthacrylates, les alkyl éthers polyoxyéthylénés ou leurs mélanges.

6. - Comprimé pelliculé selon la revendication 3, dans lequel le liant est l'hydroxypropyl méthyl cellulose ou l'hydroxypropyl cellulose.

7. - Comprimé pelliculé selon la revendication 3, dans lequel la quantité de liants est de 2,0 % à 15,0 % en poids dans la composition totale.

8. - Comprimé pelliculé selon la revendication 3, dans lequel les agents de glissement sont choisis dans le groupe comprenant le dioxyde de silicium colloïdal anhydre, le fumarate de sodium et de stéaryle, l'oxyde de magnésium, le dioxyde de silicium, le talc, le polyéthylène glycol, l'acide stéarique, le silicate d'aluminium, le silicate de magnésium ou leurs mélanges.

9. - Comprimé pelliculé selon la revendication 3, dans lequel l'agent de glissement est le dioxyde de silicium colloïdal anhydre.

10. - Comprimé pelliculé selon la revendication 1, dans lequel le comprimé pelliculé comprend :
a) de la metformine HCl
b) de l'empagliflozine
c) de l'amidon de maïs
d) de l'hydroxypropyl cellulose ou de l'hydroxypropyl méthylcellulose
e) du dioxyde de silicium colloïdal anhydre
f) du fumarate de sodium et de stéaryle.

11. - Procédé de préparation du comprimé pelliculé comprenant les étapes suivantes :
a) mélanger de la metformine HCl, de l'empagliflozine, de l'amidon de maïs et de l'hydroxypropyl cellulose ou de l'hydroxypropyl méthyl cellulose ;
b) granuler le mélange avec un solvant ;
c) sécher les granulés humides ;
d) ajouter du dioxyde de silicium colloïdal anhydre et du fumarate de sodium et de stéaryle, puis mélanger ;
e) comprimer le mélange en les comprimés ;
f) enrober les comprimés avec un agent pelliculant.

12. - Procédé selon la revendication 11, dans lequel le solvant est de l'eau.
